(19)

Europäisches Patentamt

European Patent Office

Office européen des brevets

(11) **EP 4 245 794 A1**

(12) **EUROPEAN PATENT APPLICATION**
published in accordance with Art. 153(4) EPC

(43) Date of publication:
**20.09.2023 Bulletin 2023/38**

(21) Application number: **21903240.6**

(22) Date of filing: **30.11.2021**

(51) International Patent Classification (IPC):
**C08J 3/12** *(2006.01)*          **A61F 13/53** *(2006.01)*

(52) Cooperative Patent Classification (CPC):
**A61F 13/53; C08J 3/12**

(86) International application number:
**PCT/JP2021/043854**

(87) International publication number:
**WO 2022/124136 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.12.2020   JP 2020204037**

(71) Applicant: **Sumitomo Seika Chemicals Co., Ltd.
Kako-gun, Hyogo 675-0145 (JP)**

(72) Inventors:
• **SAWAKI Hiroki
  Himeji-shi, Hyogo 672-8076 (JP)**
• **SANO Kentaro
  Himeji-shi, Hyogo 672-8076 (JP)**

(74) Representative: **Vossius & Partner
Patentanwälte Rechtsanwälte mbB
Siebertstraße 3
81675 München (DE)**

(54) **WATER-ABSORBING RESIN PARTICLES, ABSORBENT, AND ABSORBENT ARTICLE**

(57)     Water-absorbent resin particles have polymer particles; and a polymer disposed on at least a part of a surface of the polymer particle, in which a surface free energy is 14.0 to 33.0 mJ/m$^2$, and a fracture energy of the polymer determined from a stress-strain curve in a tensile test at a tensile rate of 200 mm/min is $2.00 \times 10^6$ J/m$^3$ or more. An absorber contains the water-absorbent resin particles. An absorbent article has the absorber.

EP 4 245 794 A1

## Description

### Technical Field

The present invention relates to water-absorbent resin particles, an absorber, an absorbent article, and the like.

### Background Art

Water-absorbent resin particles are widely used in various fields of sanitary materials such as diapers, hygiene products, and portable toilets; agricultural and horticultural materials such as water retention agents and soil improvement agents; and industrial materials such as waterproofing agents and condensation prevention agents. For example, an absorber containing water-absorbent resin particles has been used in an absorbent article for absorbing a liquid (for example, urine) having water as a main component. Patent Literature 1 below discloses water-absorbent resin particles that are used for absorbers of absorbent articles such as diapers.

### Citation List

### Patent Literature

[Patent Literature 1] Japanese Unexamined Patent Publication No. H06-345819

### Summary of Invention

### Technical Problem

After water-absorbent resin particles come into contact with a liquid to be absorbed (such as water and urine), the water-absorbent resin particles start absorption and reach a swelling state (state in which absorption is no longer possible) in a relatively short time. When the water-absorbent resin particles swell, this is likely to cause a "gel blocking phenomenon" in which the gap present between the water-absorbent resin particles is filled with the swollen gel of the water-absorbent resin particles, making a liquid difficult to pass through the gap. As a result, it becomes difficult for the liquid to diffuse, which is a factor causing liquid leakage. Meanwhile, according to the findings of the inventors of the present invention, slowing down the water absorption rate of the water-absorbent resin particles is effective in preventing the gel blocking phenomenon.

An object of one aspect of the present invention is to provide water-absorbent resin particles capable of achieving a slow water absorption rate. An object of another aspect of the present invention is to provide an absorber and an absorbent article using the water-absorbent resin particles.

### Solution to Problem

One aspect of the present invention relates to water-absorbent resin particles having polymer particles; and a polymer disposed on at least a part of a surface of the polymer particle, in which a surface free energy is 14.0 to 33.0 $mJ/m^2$, and a fracture energy of the polymer determined from a stress-strain curve in a tensile test at a tensile rate of 200 mm/min is $2.00 \times 10^6$ $J/m^3$ or more.

According to such water-absorbent resin particles, a slow water absorption rate can be achieved.

Another aspect of the present invention relates to an absorber containing the above-mentioned water-absorbent resin particles.

Still another aspect of the present invention relates to an absorbent article having the above-mentioned absorber.

### Advantageous Effects of Invention

According to one aspect of the present invention, water-absorbent resin particles capable of achieving a slow water absorption rate can be provided. According to another aspect of the present invention, an absorber and an absorbent article using the water-absorbent resin particles can be provided.

### Brief Description of Drawings

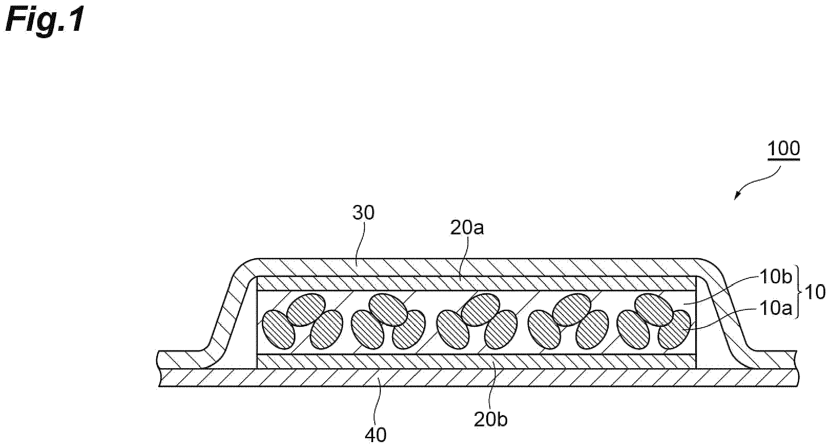

Fig. 1 is a schematic cross-sectional view showing an example of an absorbent article.

**Description of Embodiments**

Hereinafter, embodiments of the present invention will be described in detail. However, the present invention is not limited to the following embodiments, and can be variously modified and implemented within the scope of the gist thereof.

In the present specification, "acrylic" and "methacrylic" are collectively referred to as "(meth)acrylic". Similarly, "acrylate" and "methacrylate" are also referred to as "(meth)acrylate". "(Poly)" means both of a case where there is a prefix of "poly" and a case where there is no prefix thereof. In a numerical value range described in stages in the present specification, the upper limit value or the lower limit value of the numerical value range of a stage can be optionally combined with the upper limit value or the lower limit value of the numerical value range of another stage. In a numerical value range described in the present specification, the upper limit value or the lower limit value of the numerical value range may be replaced with the value shown in Examples. "Room temperature" means 25°C $\pm$ 2°C. For materials exemplified in the present specification, one type may be used alone, or two or more types may be used in combination. The content of each component in the composition means the total amount of a plurality of substances present in the composition in a case where the plurality of substances corresponding to each component are present in the composition, unless otherwise specified. "Physiological saline" means a 0.9% by mass sodium chloride aqueous solution.

Water-absorbent resin particles of the present embodiment have polymer particles, and a polymer disposed on at least a part of the surface of the polymer particle. In the water-absorbent resin particles of the present embodiment, the surface free energy is 14.0 to 33.0 mJ/m$^2$, and the fracture energy of the polymer determined from a stress-strain curve in a tensile test at a tensile rate of 200 mm/min is $2.00 \times 10^6$ J/m$^3$ or more.

According to the water-absorbent resin particles of the present embodiment, a slow water absorption rate can be achieved. The water absorption rate can be evaluated by an amount of increase in the height of a particle layer when physiological saline is brought into contact with the particle layer of the water-absorbent resin particles disposed at the bottom of a cylinder. The amount of increase in the height of the particle layer is the water absorption amount of the particle layer, and the smaller the amount of increase in the height of the particle layer is, the slower the water absorption rate is.

The following reason is presumed as the reason why a slow water absorption rate can be achieved. However, the reason why a slow water absorption rate can be achieved is not limited to the following contents.

When the surface free energy of the water-absorbent resin particles is too low, the affinity between the polymer particles and the polymer disposed on the surface of the polymer particles becomes poor, making it likely for cracks or micropores to be formed in the polymer. Due to that, water easily comes into contact with the polymer particles through the cracks or micropores, which makes it impossible to sufficiently minimize an increase of the water absorption rate. On the other hand, when the surface free energy of the water-absorbent resin particles is too high, the surface of the water-absorbent resin particles is in the excessively hydrophilic state, causing water to permeate the polymer easily. Due to that, water easily comes into contact with the polymer particles, which makes it impossible to sufficiently minimize an increase of the water absorption rate. On the other hand, when the surface free energy of the water-absorbent resin particles is in the above-mentioned range, because cracks or micropores are unlikely to be formed in the polymer, and also water does not permeate excessively quickly, water comes into contact with the polymer particles slowly, which makes it possible to achieve a slow water absorption rate.

When the fracture energy of the polymer is too low, expansion of the polymer particles caused by water absorption cannot be sufficiently reduced, making cracks or micropores likely to be formed on the surface of the polymer particles. Due to that, as in the case in which the surface free energy of the water-absorbent resin particles is too low, an increase in the water absorption rate cannot be sufficiently minimized. On the other hand, when the fracture energy of the polymer is in the above-mentioned range, cracks or micropores are unlikely to be formed in the polymer even when the polymer particles expand to some extent, and as a result, an increase in the water absorption rate is sufficiently minimized.

The surface free energy (25°C) of the water-absorbent resin particles is 14.0 to 33.0 mJ/m$^2$ from the viewpoint of achieving a slow water absorption rate. From the viewpoint of easily achieving a slow water absorption rate, the surface free energy of the water-absorbent resin particles may be 15.0 mJ/m$^2$ or more, 16.0 mJ/m$^2$ or more, 17.0 mJ/m$^2$ or more, 18.0 mJ/m$^2$ or more, or 19.0 mJ/m$^2$ or more. From the viewpoint of adjusting the water absorption rate, the surface free energy of the water-absorbent resin particles may be 20.0 mJ/m$^2$ or more, 20.5 mJ/m$^2$ or more, 21.0 mJ/m$^2$ or more, 22.0 mJ/m$^2$ or more, 25.0 mJ/m$^2$ or more, 28.0 mJ/m$^2$ or more, 30.0 mJ/m$^2$ or more, or 31.0 mJ/m$^2$ or more. From the viewpoint of easily achieving a slow water absorption rate, the surface free energy of the water-absorbent resin particles may be 32.0 mJ/m$^2$ or less, 31.0 mJ/m$^2$ or less, 30.0 mJ/m$^2$ or less, 28.0 mJ/m$^2$ or less, 25.0 mJ/m$^2$ or less, 22.0 mJ/m$^2$ or less, 21.0 mJ/m$^2$ or less, 20.5 mJ/m$^2$ or less, or 20.0 mJ/m$^2$ or less. From the viewpoint of adjusting the water absorption rate, the surface free energy of the water-absorbent resin particles may be 19.0 mJ/m$^2$ or less, 18.0 mJ/m$^2$ or less, 17.0 mJ/m$^2$ or less, or 16.0 mJ/m$^2$ or less. From these viewpoints, the surface free energy of the water-absorbent resin particles may be 15.0 to 32.0 mJ/m$^2$, 15.0 to 30.0 mJ/m$^2$, 15.0 to 25.0 mJ/m$^2$, 15.0 to 22.0 mJ/m$^2$, 15.0

to 20.0 mJ/m$^2$, 15.0 to 19.0 mJ/m$^2$, 16.0 to 32.0 mJ/m$^2$, 16.0 to 30.0 mJ/m$^2$, 16.0 to 25.0 mJ/m$^2$, 16.0 to 22.0 mJ/m$^2$, 16.0 to 20.0 mJ/m$^2$, 17.0 to 22.0 mJ/m$^2$, 19.0 to 32.0 mJ/m$^2$, 19.0 to 30.0 mJ/m$^2$, 19.0 to 25.0 mJ/m$^2$, 19.0 to 22.0 mJ/m$^2$, 19.0 to 20.0 mJ/m$^2$, 20.0 to 32.0 mJ/m$^2$, 20.0 to 30.0 mJ/m$^2$, 20.0 to 25.0 mJ/m$^2$, or 20.0 to 22.0 mJ/m$^2$. The surface free energy can be calculated by the Kaelble-Uy method based on the contact angle (25°C) with respect to ion-exchanged water and formamide obtained using a contact angle meter (manufactured by Kyowa Interface Science Co., Ltd., trade name: DMo-601). The surface free energy can be adjusted by selecting a material to be used, processing fine unevenness, and the like.

The fracture energy (fracture toughness value, 25°C) of the polymer (polymer disposed on at least a part of the surface of the polymer particles) which is determined from the stress-strain curve in a tensile test at a tensile rate of 200 mm/min is $2.00 \times 10^6$ J/m$^3$ or more from the viewpoint of achieving a slow water absorption rate. From the viewpoint of easily achieving a slow water absorption rate, the fracture energy may be $3.00 \times 10^6$ J/m$^3$ or more, $5.00 \times 10^6$ J/m$^3$ or more, $6.00 \times 10^6$ J/m$^3$ or more, $7.00 \times 10^6$ J/m$^3$ or more, $8.00 \times 10^6$ J/m$^3$ or more, $1.00 \times 10^7$ J/m$^3$ or more, $2.00 \times 10^7$ J/m$^3$ or more, $2.50 \times 10^7$ J/m$^3$ or more, $2.55 \times 10^7$ J/m$^3$ or more, or $2.60 \times 10^7$ J/m$^3$ or more. From the viewpoint of adjusting the water absorption rate, the fracture energy may be $3.00 \times 10^7$ J/m$^3$ or more, $5.00 \times 10^7$ J/m$^3$ or more, $8.00 \times 10^7$ J/m$^3$ or more, $1.00 \times 10^8$ J/m$^3$ or more, or $1.50 \times 10^8$ J/m$^3$ or more. From the viewpoint that excessive reduction of the expansion (water absorption) of the polymer particles is unlikely to occur, the fracture energy may be $1.80 \times 10^8$ J/m$^3$ or less, $1.60 \times 10^8$ J/m$^3$ or less, $1.50 \times 10^8$ J/m$^3$ or less, $1.00 \times 10^8$ J/m$^3$ or less, $8.00 \times 10^7$ J/m$^3$ or less, $5.00 \times 10^7$ J/m$^3$ or less, $3.00 \times 10^7$ J/m$^3$ or less, or $2.60 \times 10^7$ J/m$^3$ or less. From the viewpoint of adjusting the water absorption rate, the fracture energy may be $2.55 \times 10^7$ J/m$^3$ or less, $2.50 \times 10^7$ J/m$^3$ or less, $2.00 \times 10^7$ J/m$^3$ or less, $1.00 \times 10^7$ J/m$^3$ or less, $8.00 \times 10^6$ J/m$^3$ or less, or $7.00 \times 10^6$ J/m$^3$ or less. From these viewpoints, the fracture energy may be $2.00 \times 10^6$ to $1.80 \times 10^8$ J/m$^3$, $3.00 \times 10^6$ to $1.80 \times 10^8$ J/m$^3$, $1.00 \times 10^7$ to $1.80 \times 10^8$ J/m$^3$, $2.50 \times 10^7$ to $1.80 \times 10^8$ J/m$^3$, $2.60 \times 10^7$ to $1.80 \times 10^8$ J/m$^3$, $5.00 \times 10^7$ to $1.80 \times 10^8$ J/m$^3$, $1.00 \times 10^8$ to $1.80 \times 10^8$ J/m$^3$, $2.00 \times 10^6$ to $1.60 \times 10^8$ J/m$^3$, $3.00 \times 10^6$ to $1.60 \times 10^8$ J/m$^3$, $1.00 \times 10^7$ to $1.60 \times 10^8$ J/m$^3$, $2.50 \times 10^7$ to $1.60 \times 10^8$ J/m$^3$, $2.60 \times 10^7$ to $1.60 \times 10^8$ J/m$^3$, $5.00 \times 10^7$ to $1.60 \times 10^8$ J/m$^3$, $1.00 \times 10^8$ to $1.60 \times 10^8$ J/m$^3$, $2.00 \times 10^6$ to $1.00 \times 10^8$ J/m$^3$, $3.00 \times 10^6$ to $1.00 \times 10^8$ J/m$^3$, $1.00 \times 10^7$ to $1.00 \times 10^8$ J/m$^3$, $2.50 \times 10^7$ to $1.00 \times 10^8$ J/m$^3$, or $2.60 \times 10^7$ to $1.00 \times 10^8$ J/m$^3$.

The fracture energy can be determined as an integration value of the stress-strain curve (vertical axis: stress, horizontal axis: strain) which is obtained by performing a tensile test at a tensile rate of 200 mm/min using a test piece after obtaining the test piece by cutting it out of a polymer film of the same polymer as the polymer disposed on at least a part of the surface of the polymer particles using a dumbbell cutter (manufactured by DUMBBELL CO., LTD., SDMP-1000). The fracture energy varies depending on the type of polymer, and in the same type of polymer, the larger the molecular weight, the higher the fracture energy.

The water retention amount (25°C) of physiological saline of the water-absorbent resin particles may be in the following range. The water retention amount may be 10 g/g or more, 15 g/g or more, 20 g/g or more, 25 g/g or more, more than 25 g/g, 28 g/g or more, 29 g/g or more, 30 g/g or more, more than 30 g/g, 35 g/g or more, more than 35 g/g, 38 g/g or more, 38.5 g/g or more, 38.8 g/g or more, 39 g/g or more, 40 g/g or more, 41 g/g or more, 42 g/g or more, or 43 g/g or more. The water retention amount may be 100 g/g or less, 90 g/g or less, 80 g/g or less, 70 g/g or less, 60 g/g or less, 55 g/g or less, 50 g/g or less, 45 g/g or less, 44 g/g or less, 43 g/g or less, 42 g/g or less, 41 g/g or less, 40 g/g or less, 39 g/g or less, 38.8 g/g or less, 38.5 g/g or less, 38 g/g or less, 35 g/g or less, less than 35 g/g, 30 g/g or less, less than 30 g/g, or 29 g/g or less. From these viewpoints, the water retention amount may be 10 to 100 g/g, 20 to 100 g/g, 25 to 100 g/g, 30 to 100 g/g, 40 to 100 g/g, 10 to 70 g/g, 20 to 70 g/g, 25 to 70 g/g, 30 to 70 g/g, 40 to 70 g/g, 10 to 50 g/g, 20 to 50 g/g, 25 to 50 g/g, 30 to 50 g/g, 40 to 50 g/g, 10 to 40 g/g, 20 to 40 g/g, or 25 to 40 g/g. The water retention amount can be measured by a method described in Examples to be described later.

The medium particle diameter (25°C) of the water-absorbent resin particles of the present embodiment may be in the following range. The medium particle diameter of the water-absorbent resin particles may be 100 $\mu$m or more, 150 $\mu$m or more, 200 $\mu$m or more, 250 $\mu$m or more, 300 $\mu$m or more, 340 $\mu$m or more, 345 $\mu$m or more, 350 $\mu$m or more, 351 $\mu$m or more, 352 $\mu$m or more, or 355 $\mu$m or more. The medium particle diameter of the water-absorbent resin particles may be 800 $\mu$m or less, 700 $\mu$m or less, 600 $\mu$m or less, 500 $\mu$m or less, 450 $\mu$m or less, 420 $\mu$m or less, 400 $\mu$m or less, less than 400 $\mu$m, 380 $\mu$m or less, 370 $\mu$m or less, or 360 $\mu$m or less. From these viewpoints, the medium particle diameter of the water-absorbent resin particles may be 100 to 800 $\mu$m, 100 to 500 $\mu$m, 100 to 400 $\mu$m, 100 to 360 $\mu$m, 300 to 800 $\mu$m, 300 to 500 $\mu$m, 300 to 400 $\mu$m, or 300 to 360 $\mu$m. The medium particle diameter may be a particle diameter based on mass. The medium particle diameter can be measured by a method described in Examples to be described below.

The polymer particles (bodies to be coated) in the water-absorbent resin particles of the present embodiment may have water absorption properties. The shape of the polymer particles is not particularly limited, and may be substantially spherical, amorphous, granular, or the like, and may be a shape in which primary particles having these shapes are aggregated, for example. The amorphous polymer particles can be obtained by crushing the mass of the polymer with a crusher, for example.

The polymer particles may be surface-crosslinked or may not be surface-crosslinked. In the surface-crosslinked polymer particles, the crosslinking density on the surface is higher than that on the inside of the particles.

The polymer particles may contain a gel stabilizer, a metal chelating agent, a flowability improver (lubricant), or the like. These components may be disposed inside the polymer particles, or on the surface of the polymer particles, or both inside and on the surface thereof.

The polymer particles can have an ethylenically unsaturated monomer as a monomer unit (can have a structural unit derived from an ethylenically unsaturated monomer). The polymer particles can also have a monomer different from the ethylenically unsaturated monomer as a monomer unit.

The ethylenically unsaturated monomer may be a water-soluble ethylenically unsaturated monomer (for example, an ethylenically unsaturated monomer having the solubility of 1 g or more in 100 g of ion-exchanged water at 25°C). Examples of the ethylenically unsaturated monomer include (meth)acrylic acid and salts thereof, (meth)acrylic acid esters (methyl (meth)acrylate, ethyl (meth)acrylate, 2-hydroxyethyl (meth)acrylate, 2-(diethylamino)ethyl (meth)acrylate, 2-(diethylamino)propyl (meth)acrylate, and the like), (meth)acrylamide-based monomers ((meth)acrylamide, N-isopropyl(meth)acrylamide, 2-(meth)acrylamide-2-methylpropanesulfonic acid and salts thereof, N,N-dimethyl(meth)acrylamide, N-methylol(meth)acrylamide, diethylaminopropyl (meth)acrylamide, and the like), and polyethylene glycol mono(meth)acrylate. From the viewpoint of easily achieving a slow water absorption rate, the ethylenically unsaturated monomer may include at least one (meth)acrylic acid component selected from the group consisting of (meth)acrylic acid and a salt thereof, and the polymer particles may have at least one (meth)acrylic acid component selected from the group consisting of (meth)acrylic acid and a salt thereof as a monomer unit.

The content of the monomer unit of the ethylenically unsaturated monomer may be 70 to 100 mol% based on the total amount of the monomer units constituting the polymer particles. The content of the monomer unit of the (meth)acrylic acid component (total amount of the monomer unit of the (meth)acrylic acid and a salt thereof) may be 70 to 100 mol% based on the total amount of the monomer units constituting the polymer particles.

The polymer disposed on the surface of the polymer particles can prevent the polymer particles from coming into contact with a liquid to be absorbed. The polymer disposed on the surface of the polymer particles may form a cover part (coating material) that covers at least a part of the surface of the polymer particles (bodies to be coated) (where the water-absorbent resin particles may be coated resin particles having polymer particles and a cover part). It is sufficient for the cover part to cover at least a part of the surface of the polymer particle, and the cover part covers a part or the entire part of the surface of the polymer particle. The cover part may have a one-layer structure or a multilayer structure having two or more layers.

The polymer disposed on the surface of the polymer particles may be different from the polymer constituting the polymer particles. The polymer disposed on the surface of the polymer particles may be water-soluble or may not be water-soluble (may be poorly water-soluble). The polymer may contain a water-soluble component or may contain a poorly water-soluble component. The term "water-soluble" means a solubility of 1 g or more (for example, 1 to 150 g) in 100 g of ion-exchanged water at 25°C. The term "poorly water-soluble" means a solubility of less than 1 g in 100 g of ion-exchanged water at 25°C.

The water-soluble component may include a compound having a hydrophilic group. Examples of the hydrophilic group include an anionic group, a cationic group, an amphoteric group, and a nonionic group. Examples of the anionic group include a carboxyl group, a sulfonic acid group, and a phosphoric acid group. Examples of the cationic group include an amino group, an imino group, and a quaternary ammonium group. Examples of the amphoteric group include a carbobetaine group, a sulfobetaine group, and a phosphobetaine group. Examples of the nonionic group include a hydroxyl group; an amide group; a cyclic lactam group such as a pyrrolidone group and a caprolactam group; an alkoxy group; and a (poly)oxyalkylene group such as a (poly)oxyethylene group and a (poly)oxypropylene group.

Examples of compounds having a hydroxyl group include polyvinyl alcohols. Examples of compounds having an amide group include polyacrylamide. Examples of compounds having a (poly)oxyalkylene group include polyalkylene oxides (for example, polyethylene oxide) and polyalkylene glycols (for example, polyethylene glycol).

As the water-insoluble component, a water-insoluble organic compound, a water-insoluble inorganic compound, or the like can be used.

Examples of the water-insoluble organic compound include polyoxyalkylene alkyl ether (for example, polyoxyethylene alkyl ethers such as polyoxyethylene oleyl ether, polyoxyethylene lauryl ether, and polyoxyethylene stearyl ether); polyesters such as polyethylene terephthalate, polybutylene terephthalate, polyethylene naphthalate, and polybutylene naphthalate; polyamides such as nylon 6 and nylon 66; polyolefins such as polyethylene, polypropylene, ethylene-butene copolymers, and ethylene-propylene copolymers; polyurethanes such as ether-based polyurethanes, ester-based polyurethanes, and carbonate-based polyurethanes; polystyrene such as poly-α-methylstyrene and syndiotactic polystyrene; bisphenol A; polycarbonates such as polyhexamethylene carbonate; poly(meth)acrylates such as trimethylolpropane tri(meth)acrylate, pentaerythritol tri(meth)acrylate, dipentaerythritol hexa(meth)acrylate; polyalkyl (meth)acrylates such as polymethyl (meth)acrylate; polyacetals such as polyoxymethylene, polyacetaldehyde, polypropionaldehyde, and polybutyraldehyde; vinyl halide polymers such as polyvinyl chloride and polyvinyl fluoride; polyvinylidene fluoride; polysiloxane; rubber components such as styrene-butadiene rubber, nitrile rubber, acrylic rubber, polybutadiene rubber, butyl rubber, chloroprene rubber, polyisoprene rubber, and polysulfide rubber.

Polyurethane is a reaction product of polyol and polyisocyanate. The polyol may be any compound as long as it has two or more hydroxyl groups, and diols, triols, and the like can be used. Examples of the polyols include polyether polyols, polyester polyols, polycarbonate polyols, polysiloxane polyols, polyisoprene polyols, polyolefin polyols, polybutadiene polyol, and hydrogenated polybutadiene polyols. The polyisocyanate may be any compound as long as it has two or more isocyanate groups, and diisocyanate, triisocyanate, and the like can be used. Examples of the polyisocyanates include aromatic isocyanates such as diphenylmethane diisocyanate, dimethyldiphenylmethane diisocyanate, tolylene diisocyanate (for example, tolylene-2,4-diisocyanate), xylylene diisocyanate, and p-phenylene diisocyanate; alicyclic isocyanates such as dicyclohexylmethane diisocyanate and isophorone diisocyanate; and aliphatic isocyanates such as hexamethylene diisocyanate.

The polymer disposed on the surface of the polymer particles may contain a reaction product of aldehydes and phenolic compounds; a reaction product of polyols and polycarboxylic acids; a reaction product of polyamines and polycarboxylic acids; a reaction product of phenolic compounds and carbonate esters; and a reaction product of phenolic compounds and carbonate chlorides. Examples of the aldehydes include aliphatic aldehydes such as formaldehyde, acetaldehyde, and propionaldehyde; and aromatic aldehydes such as benzaldehyde. Examples of the phenolic compounds include phenol, cresol, catechol, naphthol, and hydroquinone.

The polymer disposed on the surface of the polymer particles may include a polymer having an ethylenically unsaturated monomer as a monomer unit (a polymer having a structural unit derived from an ethylenically unsaturated monomer). Examples of the ethylenically unsaturated monomer include (meth)acrylic acid and salts thereof, (meth)acrylic acid esters (methyl (meth)acrylate, ethyl (meth)acrylate, 2-hydroxyethyl (meth)acrylate, 2-(diethylamino)ethyl (meth)acrylate, 2-(diethylamino)propyl (meth)acrylate, and the like), (meth)acrylamide-based monomers ((meth)acrylamide, N-isopropyl(meth)acrylamide, 2-(meth)acrylamide-2-methylpropanesulfonic acid and salts thereof, N,N-dimethyl(meth)acrylamide, N-methylol(meth)acrylamide, diethylaminopropyl (meth)acrylamide, and the like), and polyethylene glycol mono(meth)acrylate.

As the polymer disposed on the surface of the polymer particles, it is possible to use a chain polymerization reaction product such as poly(meth)acrylic acid, poly(meth)acrylamide, polyvinyl alcohol, polyalkylene oxide, and polyalkylene glycol; and a sequential polymerization reaction product such as polyurethane (urethane resin), phenolic resin (for example, a condensate of a phenolic compound and an aldehyde), polyester, polyamide, polycarbonate; or the like.

The water-insoluble organic compound may be acid-modified. The water-insoluble organic compound may be acid-modified with an acid anhydride (such as maleic acid anhydride, succinic acid anhydride, and phthalic acid anhydride), for example.

Examples of the water-insoluble inorganic compound include light anhydrous silicic acid, calcium silicate, silicon dioxide, talc, silicon oxide, and synthetic hydrotalcite.

From the viewpoint of easily achieving a slow water absorption rate, the polymer disposed on the surface of the polymer particles may contain a polyvinyl alcohol, may contain a polyalkylene oxide (for example, polyethylene oxide), may contain a polyalkyl (meth)acrylate (for example, polymethyl (meth)acrylate), or may contain polyvinyl chloride.

A method for producing water-absorbent resin particles of the present embodiment includes a water-absorbent resin particle production step of disposing the polymer on at least a part of the surface of the polymer particles. The water-absorbent resin particle production step may be a polymerization step of obtaining the polymer by polymerizing monomers on at least a part of the surface of the polymer particles, or may be a step of bringing the polymer obtained by preliminarily polymerizing monomers into contact with at least a part of the surface of the polymer particles. The polymerization reaction in the polymerization step may be a chain polymerization reaction, a sequential polymerization reaction, or the like.

The method for producing water-absorbent resin particles of the present embodiment may include a surface crosslinking step of surface-crosslinking the polymer particles before the water-absorbent resin particle production step. In the surface crosslinking step, the polymer particles can be surface-crosslinked by mixing the polymer particles and a surface crosslinking agent. Examples of the surface crosslinking agent include polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether.

The method for producing water-absorbent resin particles of the present embodiment may include a polymer particle production step of polymerizing monomers to obtain the polymer particles before the surface crosslinking step and the water-absorbent resin particle production step. In the polymer particle production step, the monomer can be polymerized once or multiple times.

For example, the polymer particles can be obtained by polymerizing a monomer including an ethylenically unsaturated monomer, and can also be obtained by polymerizing an ethylenically unsaturated monomer and a monomer other than an ethylenically unsaturated monomer. Examples of polymerization methods of an ethylenically unsaturated monomer include a reverse phase suspension polymerization method, an aqueous solution polymerization method, a bulk polymerization method, and a precipitation polymerization method.

When the ethylenically unsaturated monomer has an acid group, the acid group may be neutralized and then used in the polymerization reaction. The degree of neutralization in the ethylenically unsaturated monomer may be 10 to 100 mol%, 50 to 90 mol%, or 60 to 80 mol% of the acid group in the ethylenically unsaturated monomer.

An internal crosslinking agent may be used for obtaining the polymer particles. When the internal crosslinking agent is used in the polymerization of the monomer, a crosslinking density is substantially uniformly increased easily in almost all of the polymer particles. Examples of the internal crosslinking agent include polyglycidyl compounds such as (poly)ethylene glycol diglycidyl ether, (poly)propylene glycol diglycidyl ether, (poly)glycerin diglycidyl ether, (poly)glycerin triglycidyl ether, (poly)propylene glycol polyglycidyl ether, and polyglycerol polyglycidyl ether; divinyl-based compounds; dialcohol-based compounds; and diacrylate-based compounds.

The method for producing water-absorbent resin particles of the present embodiment may include a step of classifying the water-absorbent resin particles by a sieve after the water-absorbent resin particle production step. This makes it possible to adjust particle size distribution.

An absorber of the present embodiment contains the water-absorbent resin particles of the present embodiment. The absorber of the present embodiment may contain a fibrous substance, and is a mixture containing the water-absorbent resin particles and the fibrous substance, for example. For example, the structure of the absorber may be a structure in which the water-absorbent resin particles and the fibrous substance are uniformly mixed, may be a structure in which the water-absorbent resin particles are sandwiched between the fibrous substances formed in the shape of a sheet or a layer, or may be other structures.

Examples of the fibrous substance include finely pulverized wood pulp; cotton; cotton linter; rayon; cellulosic fibers such as cellulose acetate; synthetic fibers such as polyamide, polyester, and polyolefin; and a mixture of these fibers. The fibrous substance may be used alone, or may be used in combination of two or more types. As the fibrous substance, hydrophilic fibers can be used.

In order to enhance the morphological retention before and during use of the absorber, the fibers may be adhered to each other by adding an adhesive binder to the fibrous substance. Examples of the adhesive binder include thermal bonding synthetic fibers, hot melt adhesives, and adhesive emulsions. The adhesive binder may be used alone, or may be used in combination of two or more types.

Examples of the thermal bonding synthetic fibers include a total fusion type binder such as polyethylene, polypropylene, and an ethylene-propylene copolymer; and a non-total fusion type binder made of a side-by-side or core-sheath structure of polypropylene and polyethylene. In the above-mentioned non-total fusion type binder, only the polyethylene portion can be thermal-bonded.

Examples of the hot melt adhesives include a mixture of a base polymer such as ethylene-vinyl acetate copolymer, styrene-isoprene-styrene block copolymer, styrene-butadiene-styrene block copolymer, styrene-ethylene-butylene-styrene block copolymer, styrene-ethylene-propylene-styrene block copolymer, and amorphous polypropylene with a tackifier, a plasticizer, an antioxidant, or the like.

Examples of the adhesive emulsions include a polymerization product of at least one monomer selected from the group consisting of methyl methacrylate, styrene, acrylonitrile, 2-ethylhexyl acrylate, butyl acrylate, butadiene, ethylene, and vinyl acetate.

The absorber of the present embodiment may contain an inorganic powder (for example, amorphous silica), a deodorant, an antibacterial agent, a dye, a pigment, a fragrance, a sticking agent, or the like. When the water-absorbent resin particles contain inorganic particles, the absorber may contain an inorganic powder in addition to the inorganic particles of the water-absorbent resin particles.

The shape of the absorber of the present embodiment may be a sheet shape, for example. The thickness of the absorber (for example, the thickness of the sheet-shaped absorber) may be 0.1 to 20 mm, or 0.3 to 15 mm.

The content of the water-absorbent resin particles in the absorber may be in the following range based on the total mass of the absorber or the total amount of the water-absorbent resin particles and the fibrous substance. The content of the water-absorbent resin particles may be 2% by mass or more, 10% by mass or more, 20% by mass or more, or 50% by mass or more from the viewpoint of easily obtaining sufficient water-absorbent characteristics. The content of the water-absorbent resin particles is 100% by mass or less, and from the viewpoint of easily obtaining sufficient water-absorbent characteristics, the content may be 80% by mass or less, 70% by mass or less, or 60% by mass or less. From these viewpoints, the content of the water-absorbent resin particles may be 2% to 100% by mass, 10% to 80% by mass, 20% to 70% by mass, or 50% to 60% by mass.

An absorbent article of the present embodiment has the absorber of the present embodiment. Examples of other constituent members of the absorbent article of the present embodiment include a core wrap that retains the shape of the absorber and prevents falloff or flow of the constituent member of the absorber; a liquid permeable sheet disposed on the outermost part at the side where a liquid to be absorbed enters; and a liquid impermeable sheet disposed on the outermost part at the opposite side to the side where the liquid to be absorbed enters. Examples of the absorbent article include diapers (for example, disposable diapers), toilet training pants, incontinence pads, sanitary materials (sanitary napkins, tampons, and the like), sweat pads, pet sheets, portal toilet members, and animal excrement treatment materials.

Fig. 1 is a cross-sectional view showing an example of the absorbent article. An absorbent article 100 shown in Fig. 1 has an absorber 10, core wraps 20a and 20b, a liquid permeable sheet 30, and a liquid impermeable sheet 40. In the absorbent article 100, the liquid impermeable sheet 40, the core wrap 20b, the absorber 10, the core wrap 20a, and the liquid permeable sheet 30 are laminated in this order. In Fig. 1, there is a portion shown so that there is a gap between the members, but the members may be in close contact with each other without the gap.

The absorber 10 has water-absorbent resin particles 10a of the present embodiment and a fiber layer 10b containing a fibrous substance. The water-absorbent resin particles 10a are dispersed in the fiber layer 10b.

The core wrap 20a is disposed on one surface side of the absorber 10 (upper side of the absorber 10 in Fig. 1) in the state of being in contact with the absorber 10. The core wrap 20b is disposed on the other surface side of the absorber 10 (lower side of the absorber 10 in Fig. 1) in the state of being in contact with the absorber 10. The absorber 10 is disposed between the core wrap 20a and the core wrap 20b. Examples of the core wraps 20a and 20b include tissues, non-woven fabrics, woven fabrics, synthetic resin films having liquid permeation holes, and net-like sheets having a mesh. The core wrap 20a and the core wrap 20b each have a main surface having the same size as that of the absorber 10, for example.

The liquid permeable sheet 30 is disposed on the outermost part at the side where the liquid to be absorbed enters. The liquid permeable sheet 30 is disposed on the core wrap 20a in the state of being in contact with the core wrap 20a. Examples of the liquid permeable sheet 30 include a non-woven fabric and a porous sheet which are made of a synthetic resin such as polyethylene, polypropylene, polyester, and polyamide. The liquid impermeable sheet 40 is disposed on the outermost part at the opposite side to the liquid permeable sheet 30 in the absorbent article 100. The liquid impermeable sheet 40 is disposed on the lower side of the core wrap 20b in the state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. For example, the liquid permeable sheet 30 and the liquid impermeable sheet 40 have a main surface wider than the main surface of the absorber 10, and outer edges of the liquid permeable sheet 30 and the liquid impermeable sheet 40 are present around the absorber 10 and the core wraps 20a and 20b.

The magnitude relationship between the absorber 10, the core wraps 20a and 20b, the liquid permeable sheet 30, and the liquid impermeable sheet 40 is not particularly limited and is appropriately adjusted according to the usage and the like of the absorbent article. In addition, a method of retaining the shape of the absorber 10 using the core wraps 20a and 20b is not particularly limited, and the absorber may be wrapped by a plurality of the core wraps as shown in Fig. 1, and the absorber may be wrapped by one core wrap.

The absorber may be adhered to a top sheet. In a case where the absorber is sandwiched between or covered by the core wraps, at least the core wrap and the top sheet may be adhered to each other, and the core wrap and the absorber may be adhered to each other while the core wrap and the top sheet are also adhered to each other. Examples of methods of adhering the absorber include a method of adhering by applying a hot melt adhesive to the top sheet at predetermined intervals in a striped shape, a spiral shape, or the like in a width direction; and a method of adhering using a water-soluble binder such as starch, carboxymethyl cellulose, polyvinyl alcohol, polyvinylpyrrolidone, and other water-soluble polymers. In addition, in a case where the absorber contains thermal bonding synthetic fibers, a method of adhering by thermal bonding of the thermal bonding synthetic fibers may be adopted.

According to the present embodiment, a liquid absorbing method using the water-absorbent resin particles, the absorber, or the absorbent article of the present embodiment can be provided. The liquid absorbing method of the present embodiment includes a step of bringing a liquid to be absorbed into contact with the water-absorbent resin particles, the absorber, or the absorbent article of the present embodiment.

According to the present embodiment, a method for producing an absorber by using the water-absorbent resin particles obtained by the above-mentioned method for producing water-absorbent resin particles can be provided. The method for producing an absorber of the present embodiment includes a particle production step of obtaining the water-absorbent resin particles by the above-mentioned method for producing water-absorbent resin particles. The method for producing an absorber of the present embodiment may include a step of mixing the water-absorbent resin particles and the fibrous substance after the particle production step. According to the present embodiment, a method for producing an absorbent article using the absorber obtained by the above-mentioned method for producing an absorber can be provided. The method for producing an absorbent article of the present embodiment includes an absorber production step of obtaining the absorber by the above-mentioned method for producing an absorber. The method for producing an absorbent article of the present embodiment may include, after the absorber production step, a step of obtaining the absorbent article by using the absorber and other constituent member for the absorbent article, and in this step, for example, the absorbent article is obtained by laminating the absorber and the other constituent member for the absorbent article with each other.

**Examples**

Hereinafter, contents of the present invention will be further described using Examples and Comparative examples, but the present invention is not limited to the following Examples. In the following description, when the temperature at the time of the experimental operation is not described, the experimental operation can be performed at room temperature.

<Production of particles for evaluation>

A round-bottomed cylindrical separable flask with the inner diameter of 11 cm and the internal volume of 2 L equipped with a reflux cooling device, a dropping funnel, a nitrogen gas introduction tube, and a stirrer (a stirrer blade having two stages of four inclined paddle blades having the blade diameter of 5 cm) was prepared. To this separable flask, 293 g of n-heptane and 0.736 g of a maleic acid anhydride-modified ethylene-propylene copolymer (dispersant, manufactured by Mitsui Chemicals, Inc., Hi-Wax 1105A) were added to obtain a mixture. After dissolving the dispersant in n-heptane by raising the temperature to 80°C while stirring this mixture, the mixture was cooled to 50°C.

Subsequently, 92.0 g (acrylic acid: 1.03 mol) of an aqueous solution of 80.5% by mass acrylic acid was put to a beaker having the internal volume of 300 mL. Subsequently, while cooling from the outside, 147.7 g of an aqueous solution of 20.9% by mass sodium hydroxide was added dropwise to the beaker to neutralize 75 mol% of acrylic acid. Thereafter, 0.092 g of hydroxyethyl cellulose (thickener, manufactured by Sumitomo Seika Chemicals Co., Ltd., HEC AW-15F), 0.0736 g (0.272 mmol) of potassium persulfate (water-soluble radical polymerization initiator), and 0.010 g (0.057 mmol) of ethylene glycol diglycidyl ether (internal crosslinking agent) were added and then dissolved to prepare a first stage monomer aqueous solution.

Then, the above-mentioned first stage monomer aqueous solution was added to the above-mentioned separable flask, and thereafter stirring was performed for 10 minutes. Thereafter, a reaction solution was obtained by adding, to the separable flask, a surfactant solution obtained by dissolving 0.736 g of sucrose stearic acid ester (surfactant, manufactured by Mitsubishi-Chemical Foods Corporation, Ryoto Sugar Ester S-370, HLB: 3) in 6.62 g of n-heptane. Then, the inside of the system was sufficiently replaced with nitrogen while stirring the reaction solution at the rotation speed of 550 rpm of the stirrer. Thereafter, the separable flask was immersed in a water bath at 70°C to raise the temperature of the reaction solution, and a polymerization reaction was advanced for 60 minutes to obtain a first stage polymerization slurry solution.

Subsequently, 128.8 g (acrylic acid: 1.44 mol) of an aqueous solution of 80.5% by mass acrylic acid was put to another beaker having the internal volume of 500 mL. Subsequently, while cooling from the outside, 159.0 g of an aqueous solution of 27% by mass sodium hydroxide was added dropwise to the beaker to neutralize 75 mol% of acrylic acid. Thereafter, 0.103 g (0.381 mmol) of potassium persulfate (water-soluble radical polymerization initiator) and 0.0116 g (0.067 mmol) of ethylene glycol diglycidyl ether (internal crosslinking agent) were added to the beaker containing the aqueous solution of acrylic acid, and then dissolved to prepare a second stage monomer aqueous solution.

While stirring at the rotation speed of 1000 rpm of the stirrer, the first stage polymerization slurry solution in the above-mentioned separable flask was cooled to 25°C, and then the total amount of the above-mentioned second stage monomer aqueous solution was added to the first stage polymerization slurry solution to obtain a reaction solution. After replacing the inside of the separable flask with nitrogen for 30 minutes, the separable flask was immersed in a water bath at 70°C again to raise the temperature of the reaction solution, and a second stage polymerization reaction was performed for 60 minutes to obtain a hydrogel-like polymer.

Thereafter, the temperature of the above-mentioned hydrogel-like polymer was raised in an oil bath at 125°C, and 257.7 g of water was extracted to the outside of the system while refluxing n-heptane by azeotropic distillation of n-heptane and water. Subsequently, 4.42 g (0.507 mmol) of an aqueous solution of 2% by mass ethylene glycol diglycidyl ether was added to the separable flask as a surface crosslinking agent, and thereafter the mixture was maintained at 83°C for 2 hours to obtain a dispersion liquid of the resin particles after surface crosslinking.

Thereafter, the temperature of the above-mentioned dispersion liquid of the resin particles after surface crosslinking was raised in an oil bath at 125°C, and 245 g of water was extracted to the outside of the system while refluxing n-heptane by azeotropic distillation of n-heptane and water. Then, drying was performed by evaporating n-heptane at 125°C to obtain a dried product. This dried product was passed through a sieve having an opening of 850 μm to obtain 236.8 g of resin particles in the form of aggregated spherical particles.

After repeating the above operations, the resin particles were classified with a sieve having an opening of 250 μm to obtain 500 g or more of polymer particles (bodies to be coated) having a particle diameter of 250 to 850 μm.

A coating liquid was prepared by mixing 25.0 g of polyvinyl chloride (coating material, PVC, manufactured by FUJIFILM Wako Pure Chemical Corporation, trade name: Polyvinyl Chloride) and 475.0 g of tetrahydrofuran in a polybeaker having the internal volume of 1 L.

After 500.0 g of the above-mentioned polymer particles (bodies to be coated) was injected to a container of a fluid bed granulator (manufactured by POWREX CORPORATION, FD-MP-01), hot air at 40°C was blown from the lower part of the container. Subsequently, 500.0 g of the coating liquid was sprayed on the polymer particles wound up by hot air at 40°C. After spraying the coating liquid over about 90 minutes at a constant spraying speed, drying was performed with hot air at 40°C for 30 minutes. Thus, 503.3 g of water-absorbent resin particles (coated resin particles, medium particle diameter: 352 $\mu$m) having the polymer particles and the cover part (polyvinyl chloride) covering the polymer particles was obtained as particles for evaluation.

(Example 2)

Water-absorbent resin particles (coated resin particles, medium particle diameter: 347 $\mu$m) having the polymer particles and the cover part (polymethyl methacrylate) covering the polymer particles were obtained as particles for evaluation in the same manner as in Example 1 except that the coating liquid was changed to a mixed liquid of 25.0 g of polymethyl methacrylate (coating material, PMMA, manufactured by FUJIFILM Wako Pure Chemical Corporation, trade name: Methyl Methacrylate Polymer) and 225.0 g of acetone, and that the spraying time of the coating liquid was changed to about 45 minutes.

(Example 3)

Water-absorbent resin particles (coated resin particles, medium particle diameter: 355 $\mu$m) having the polymer particles and the cover part (polyvinyl alcohol) covering the polymer particles were obtained as particles for evaluation in the same manner as in Example 1 except that the coating liquid was changed to a mixed liquid of 25.0 g of polyvinyl alcohol (coating material, PVA, manufactured by Kuraray Co., Ltd., trade name: KURARAY POVAL 3-98), 332.5 g of ion-exchanged water, and 142.5 g of ethanol.

(Example 4)

Water-absorbent resin particles (coated resin particles, medium particle diameter: 340 $\mu$m) having the polymer particles and the cover part (polyvinyl alcohol) covering the polymer particles were obtained as particles for evaluation in the same manner as in Example 1 except that the coating liquid was changed to a mixed liquid of 25.0 g of polyvinyl alcohol (coating material, PVA, manufactured by Kuraray Co., Ltd., trade name: KURARAY POVAL 11-98), 332.5 g of ion-exchanged water, and 142.5 g of ethanol.

(Example 5)

Water-absorbent resin particles (coated resin particles, medium particle diameter: 351 $\mu$m) having the polymer particles and the cover part (polyethylene oxide) covering the polymer particles were obtained as particles for evaluation in the same manner as in Example 1 except that the coating liquid was changed to a mixed liquid of 25.0 g of polyethylene oxide (coating material, PEO, manufactured by Sumitomo Seika Chemicals Co., Ltd., trade name: PEO-1), 487.5 g of ion-exchanged water, and 487.5 g of ethanol, and that the spraying time of the coating liquid was changed to about 180 minutes.

(Comparative Example 1)

500.2 g of water-absorbent resin particles (coated resin particles, medium particle diameter: 366 $\mu$m) having the polymer particles and the cover part (starch) covering the polymer particles was obtained as particles for evaluation in the same manner as in Example 1 except that the coating liquid was changed to a mixed liquid of 25.0 g of starch (coating material, manufactured by Nacalai Tesque, Inc., trade name: Soluble Starch) and 475.0 g of ion-exchanged water.

(Comparative Example 2)

506.6 g of water-absorbent resin particles (coated resin particles, medium particle diameter: 331 $\mu$m) having the polymer particles and the cover part (ethylene-vinyl acetate copolymer) covering the polymer particles was obtained as particles for evaluation in the same manner as in Example 1 except that the coating liquid was changed to a mixed liquid obtained by diluting 62.5 g of an aqueous dispersion emulsion of a 40% by mass ethylene-vinyl acetate copolymer (coating material, p-(E-VAC), manufactured by Sumitomo Seika Chemicals Co., Ltd., trade name: SEPOLSION VA407) with 187.5 g of ion-exchanged water, and that the spraying time of the coating liquid was changed to about 45 minutes.

(Comparative Example 3)

510.1 g of water-absorbent resin particles (coated resin particles, medium particle diameter: 341 $\mu$m) having the polymer particles and the cover part (polyurethane) covering the polymer particles was obtained as particles for evaluation in the same manner as in Example 1 except that the coating liquid was changed to a mixed liquid obtained by diluting 71.4 g of an aqueous dispersion emulsion of 35% by mass polyurethane (coating material, PU, manufactured by DKS Co., Ltd., trade name: SUPERFLEX 210) with 428.6 g of ion-exchanged water.

(Comparative Example 4)

507.8 g of water-absorbent resin particles (coated resin particles, medium particle diameter: 339 $\mu$m) having the polymer particles and the cover part (ethylene-vinyl chloride-vinyl acetate copolymer) covering the polymer particles was obtained as particles for evaluation in the same manner as in Example 1 except that the coating liquid was changed to a mixed liquid obtained by diluting 50.0 g of an aqueous dispersion emulsion of a 50% by mass ethylene-vinyl chloride-vinyl acetate copolymer (coating material, p-(E-VC-VAC), manufactured by Sumitomo Chemical Co., Ltd., trade name: Sumikaflex 801HQ) with 450.0 g of ion-exchanged water.

(Comparative Example 5)

As particles for evaluation, polymer particles having a particle diameter of 250 to 850 $\mu$m of Example 1 were used.

<Fracture energy of coating material>

(Production of polymer film)

[Example 1]

4.00 g of polyvinyl chloride (manufactured by FUJIFILM Wako Pure Chemical Corporation, trade name: Polyvinyl Chloride) and 36.00 g of tetrahydrofuran were added to a 100 mL sample bottle, and thereafter, until the polyvinyl chloride was completely dissolved, stirring was performed with a magnetic stirrer to produce a polymer solution.
After adding 30.00 g of the polymer solution to a Teflon beaker having an inner diameter of 6.2 cm, the beaker was covered with aluminum foil as a lid. After perforating the aluminum foil, drying was performed until the mass of the polymer film was 3.28 g by heating with a hot-air dryer (manufactured by ADVANTEC, FV-320) at 40°C.

[Example 2]

4.00 g of polymethyl methacrylate (manufactured by FUJIFILM Wako Pure Chemical Corporation, trade name: Methyl Methacrylate Polymer) and 36.00 g of acetone were added to a 100 mL sample bottle, and thereafter, until the polymethyl methacrylate was completely dissolved, stirring was performed with a magnetic stirrer to produce a polymer solution.
After adding 30.00 g of the polymer solution to a Teflon beaker having an inner diameter of 6.2 cm, the beaker was left to stand at room temperature for 18 hours. Thereafter, drying was performed until the mass of the polymer film was 3.39 g by heating with a hot-air dryer (manufactured by ADVANTEC, FV-320) at 40°C.

[Example 3]

2.00 g of polyvinyl alcohol (manufactured by Kuraray Co., Ltd., trade name: KURARAY POVAL 3-98) and 18.00 g of ion-exchanged water were added to a 50 mL sample bottle, and thereafter, the sample bottle was immersed in a hot water bath heated to 80°C to completely dissolve the polyvinyl alcohol, thereby producing a polymer solution.
After adding 10.00 g of the polymer solution to a Teflon beaker having an inner diameter of 6.2 cm, the beaker was covered with aluminum foil as a lid. After perforating the aluminum foil, drying was performed until the mass of the polymer film was 1.09 g by heating with a hot-air dryer (manufactured by ADVANTEC, FV-320) at 40°C.

[Example 4]

2.00 g of polyvinyl alcohol (manufactured by Kuraray Co., Ltd., trade name: KURARAY POVAL 11-98) and 18.00 g of ion-exchanged water were added to a 50 mL sample bottle, and thereafter, the sample bottle was immersed in a hot water bath heated to 80°C to completely dissolve the polyvinyl alcohol, thereby producing a polymer solution.

After adding 10.00 g of the polymer solution to a Teflon beaker having an inner diameter of 6.2 cm, the beaker was covered with aluminum foil as a lid. After perforating the aluminum foil, drying was performed until the mass of the polymer film was 1.11 g by heating with a hot-air dryer (manufactured by ADVANTEC, FV-320) at 40°C.

[Example 5]

2.00 g of polyethylene oxide (manufactured by Sumitomo Seika Chemicals Co., Ltd., trade name: PEO-1) and 38.00 g of ion-exchanged water were added to a 50 mL sample bottle, and thereafter, until the polyethylene oxide was completely dissolved, stirring was performed with a magnetic stirrer to produce a polymer solution.

After adding 20.00 g of the polymer solution to a Teflon beaker having an inner diameter of 6.2 cm, the beaker was covered with aluminum foil as a lid. After perforating the aluminum foil, drying was performed until the mass of the polymer film was 1.01 g by heating with a hot-air dryer (manufactured by ADVANTEC, FV-320) at 40°C.

[Comparative Example 1]

2.00 g of starch (manufactured by Nacalai Tesque, Inc., trade name: Soluble Starch) and 18.00 g of ion-exchanged water were added to a 50 mL sample bottle, and thereafter, the sample bottle was immersed in a hot water bath heated to 80°C to completely dissolve the starch, thereby producing a polymer solution.

After adding 10.00 g of the polymer solution to a Teflon beaker having an inner diameter of 6.2 cm, the beaker was covered with aluminum foil as a lid. After perforating the aluminum foil, drying was performed until the mass of the polymer film was 1.00 g by heating with a hot-air dryer (manufactured by ADVANTEC, FV-320) at 40°C.

[Comparative Example 2]

3.75 g of an aqueous dispersion emulsion of a 40% by mass ethylene-vinyl acetate copolymer (manufactured by Sumitomo Seika Chemicals Co., Ltd., trade name: SEPOLSION VA407) was added to a Teflon beaker having an inner diameter of 6.2 cm, and thereafter, the beaker was covered with aluminum foil as a lid. After perforating the aluminum foil, drying was performed until the mass of the polymer film was 1.52 g by heating with a hot-air dryer (manufactured by ADVANTEC, FV-320) at 50°C.

[Comparative Example 3]

4.29 g of an aqueous dispersion emulsion of 35% by mass polyurethane (manufactured by DKS Co., Ltd., trade name: SUPERFLEX 210) was added to a Teflon beaker having an inner diameter of 6.2 cm, and thereafter, the beaker was covered with aluminum foil as a lid. After perforating the aluminum foil, drying was performed until the mass of the polymer film was 1.50 g by heating with a hot-air dryer (manufactured by ADVANTEC, FV-320) at 50°C.

[Comparative Example 4]

7.50 g of an aqueous dispersion emulsion of a 50% by mass ethylene-vinyl chloride-vinyl acetate copolymer (manufactured by Sumitomo Chemical Co., Ltd., trade name: Sumikaflex 801HQ) was added to a Teflon beaker having an inner diameter of 6.2 cm, and thereafter, the beaker was covered with aluminum foil as a lid. After perforating the aluminum foil, drying was performed until the mass of the polymer film was 3.02 g by heating with a hot-air dryer (manufactured by ADVANTEC, FV-320) at 50°C.

(Tensile test)

A test piece having dimensions conforming to JIS K 6251-7 was cut out of the polymer film using a dumbbell cutter (manufactured by DUMBBELL CO., LTD., SDMP-1000). The test piece has one end part and the other end part each having a width of 6 mm, and a linear intermediate part (length: 12 mm) having a width of 2 mm connecting the one end part and the other end part, and the total length of the test piece was 35 mm. The thickness of the intermediate part of the test piece was measured using a film thickness gauge (manufactured by TECLOCK Co., Ltd., Thickness Gauge SM112), and the cross-sectional area of the intermediate part was obtained as the product of the width 2 mm and the thickness of the intermediate part of the test piece.

A tensile test (JIS K 6251, jig: SCG-1kNA, tensile rate: 200 mm/min) was performed on this test piece using a table-top precision universal tester (manufactured by Shimadzu Corporation, Autograph AGS-X), and the stress [MPa] and the strain [%] were calculated from the following formulas. A force generated when the test piece was pulled at a tensile rate of 200 mm/min was obtained as a test force at breakage. A stress-strain curve (vertical axis: stress; horizontal axis: strain) up to a breaking point was obtained by plotting changes in stress and strain over time. The integration value of the stress-strain curve was obtained as the fracture energy [J/m$^3$]. When calculating the integration value, a value of 1/100 of the strain [%] was adopted. The results are shown in Table 1.

$$\text{Stress [MPa]} = \text{test force [N] until breaking/cross-sectional area}$$

$$\text{[mm}^2\text{] of intermediate part of test piece}$$

$$\text{Strain [\%]} = \text{(total length of intermediate part until}$$

$$\text{breakage/initial total length (12 mm) of intermediate part)} \times 100$$

<Medium particle diameter>

The medium particle diameter of the above-mentioned particles for evaluation was measured by the following procedure. The particle size distribution of 5 g of the particles for evaluation was measured using an automatic sonic sieving particle size analyzer (Robot Sifter RPS-205, manufactured by SEISHIN ENTERPRISE Co., Ltd.), JIS standard sieves having the openings of 850 $\mu$m, 710 $\mu$m, 600 $\mu$m, 500 $\mu$m, 400 $\mu$m, 300 $\mu$m, 250 $\mu$m, and 150 $\mu$m, and a tray. The relationship between the opening of the sieve and the integrated value of the mass percentage of the particles remaining on the sieve was plotted on a logarithmic probability paper by integrating the masses of the particles remaining on the sieve in the order from the one having the largest particle diameter with respect to this particle size distribution. By connecting the plots on the logarithmic probability paper with a straight line, the particle diameter corresponding to the cumulative mass percentage of 50% by mass was obtained as the medium particle diameter (25°C).

<Water retention amount>

2.00 g of the above-mentioned particles for evaluation (excluding Comparative Example 5) was put in a cotton bag stored in a 500 mL polyethylene beaker. Subsequently, 500 g of physiological saline was poured into the cotton bag, and also, the entire cotton bag was immersed in the physiological saline, and then the upper part of the cotton bag was closed with a rubber band. After leaving the cotton bag to stand in this state for 30 minutes, centrifugation (167 G) was performed for 1 minute using a centrifuge. Then, the mass WA was measured after dehydration. The same operation was performed without putting the particles for evaluation in the cotton bag, and the mass WB of the empty cotton bag was measured. The water retention amount (25°C) of the physiological saline was calculated from the following formula. The results are shown in Table 1.

$$\text{Water retention amount [g/g]} = (\text{WA} - \text{WB})/\text{mass of particles for}$$

$$\text{evaluation} (= 2.00 \text{ g})$$

<Surface free energy>

A double-sided tape (manufactured by Nitto Denko CS System Corporation, trade name: No. 5000NS) having a size of 20 mm $\times$ 20 mm was attached to a stainless steel plate having a length of 30 mm, a width of 70 mm, and a thickness of 1 mm. By sprinkling 0.2 g of the above-mentioned particles for evaluation on the double-sided tape, the particles for evaluation were adhered to the double-sided tape. By tapping the stainless steel plate to shake excess particles for evaluation down, a sample for measurement of surface free energy was obtained. For the sample for measurement, the contact angle (25°C) with respect to ion-exchanged water and formamide (Tokyo Chemical Industry Co., Ltd.) was measured using a contact angle meter (manufactured by Kyowa Interface Science Co., Ltd., trade name: DMo-601). The surface free energy [mJ/m$^2$] (25°C) of the sample for measurement was calculated using the analysis software (FAMAS) built in the contact angle meter. The results are shown in Table 1.

<Evaluation of water absorption rate>

A particle layer was formed by laying 0.200 g (exactly weighed value) of the above-mentioned particles for evaluation on the bottom of an acrylic cylinder having an inner diameter of 2.0 cm and a depth of 8.0 cm in a layered manner such that there was no gaps, and thereafter, the height $H_0$ of the particle layer was measured. Subsequently, 20 g of physiological saline was poured into the acrylic cylinder from its upper part. The measurement was started from the time when the total amount of the physiological saline was poured, and the height $H_2$ of the particle layer was read after 2 minutes to calculate the water absorption rate (increase in the height of the particle layer, 25°C) from the following formula. The results are shown in Table 1.

$$\text{Water absorption rate [cm]} = H_2 - H_0$$

[Table 1]

| | Coating material | | Water retention amount [g/g] | Surface free energy [mJ/m$^2$] | Water absorption rate [cm] |
|---|---|---|---|---|---|
| | Type | Fracture energy [J/m$^3$] | | | |
| Example 1 | PVC | $2.60 \times 10^7$ | 28.7 | 19.1 | 0.1 |
| Example 2 | PMMA | $1.03 \times 10^7$ | 38.8 | 31.8 | 2.7 |
| Example 3 | PVA | $2.54 \times 10^7$ | 38.5 | 22.2 | 3.1 |
| Example 4 | PVA | $1.55 \times 10^8$ | 40.3 | 20.5 | 1.4 |
| Example 5 | PEO | $6.48 \times 10^6$ | 43.6 | 15.4 | 2.6 |
| Comparative Example 1 | Starch | $1.30 \times 10^5$ | 37.9 | 17.6 | 3.7 |
| Comparative Example 2 | p-(E-VAC) | $1.75 \times 10^7$ | 37.8 | 13.7 | 4.1 |
| Comparative Example 3 | PU | $1.23 \times 10^6$ | 26.1 | 25.1 | 3.8 |
| Comparative Example 4 | p-(E-VC-VAC) | $3.47 \times 10^6$ | 38.7 | 33.6 | 4.2 |
| Comparative Example 5 | Not used | - | - | 19.1 | 3.5 |

**Reference Signs List**

10: absorber, 10a: water-absorbent resin particle, 10b: fiber layer, 20a and 20b: core wrap, 30: liquid permeable sheet, 40: liquid impermeable sheet, 100: absorbent article.

**Claims**

1.  Water-absorbent resin particles comprising:

    polymer particles; and
    a polymer disposed on at least a part of a surface of the polymer particle,
    wherein a surface free energy is 14.0 to 33.0 mJ/m$^2$, and
    a fracture energy of the polymer determined from a stress-strain curve in a tensile test at a tensile rate of 200 mm/min is $2.00 \times 10^6$ J/m$^3$ or more.

2.  The water-absorbent resin particles according to claim 1, wherein the fracture energy is $2.00 \times 10^6$ to $1.80 \times 10^8$ J/m$^3$.

3.  The water-absorbent resin particles according to claim 1 or 2, wherein the polymer particle has an ethylenically unsaturated monomer as a monomer unit.

4.  The water-absorbent resin particles according to claim 3, wherein the ethylenically unsaturated monomer includes at least one selected from the group consisting of (meth)acrylic acid and a salt thereof.

5. The water-absorbent resin particles according to any one of claims 1 to 4, wherein the polymer contains polyvinyl chloride.

6. The water-absorbent resin particles according to any one of claims 1 to 5, wherein the polymer contains polyalkyl (meth)acrylate.

7. The water-absorbent resin particles according to any one of claims 1 to 6, wherein the polymer contains polyvinyl alcohol.

8. The water-absorbent resin particles according to any one of claims 1 to 7, wherein the polymer contains polyalkylene oxide.

9. An absorber comprising the water-absorbent resin particles according to any one of claims 1 to 8.

10. An absorbent article comprising the absorber according to claim 9.

EP 4 245 794 A1

*Fig.1*

## INTERNATIONAL SEARCH REPORT

| International application No. |
| --- |
| **PCT/JP2021/043854** |

**A.    CLASSIFICATION OF SUBJECT MATTER**

*C08J 3/12*(2006.01)i; *A61F 13/53*(2006.01)i
FI:    C08J3/12 Z; A61F13/53 300

According to International Patent Classification (IPC) or to both national classification and IPC

**B.    FIELDS SEARCHED**

Minimum documentation searched (classification system followed by classification symbols)

C08J3/00-3/28; C08J99/00; A61F13/53

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 11-347403 A (SANYO CHEM IND LTD) 21 December 1999 (1999-12-21) claims, paragraphs [0004], [0020]-[0024], examples | 1-4, 7, 9-10 |
| A | | 5-6, 8 |
| A | JP 2014-198853 A (EVONIK CORPORATION) 23 October 2014 (2014-10-23) entire text, all drawings | 1-10 |
| A | WO 2011/136301 A1 (NIPPON SHOKUBAI CO., LTD.) 03 November 2011 (2011-11-03) entire text, all drawings | 1-10 |
| A | JP 2007-77393 A (NIPPON SHOKUBAI CO., LTD.) 29 March 2007 (2007-03-29) entire text, all drawings | 1-10 |

☐ Further documents are listed in the continuation of Box C.    ☑ See patent family annex.

| | |
| --- | --- |
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **27 January 2022** | **08 February 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/043854**

| Patent document cited in search report | | | Publication date (day/month/year) | Patent family member(s) | | | Publication date (day/month/year) |
|---|---|---|---|---|---|---|---|
| JP | 11-347403 | A | 21 December 1999 | (Family: none) | | | |
| JP | 2014-198853 | A | 23 October 2014 | US | 2008/0234420 | A1 | |
| | | | | claims, examples, figures | | | |
| | | | | EP | 2137240 | A1 | |
| | | | | CN | 101679648 | A | |
| | | | | TW | 200906923 | A | |
| WO | 2011/136301 | A1 | 03 November 2011 | US | 2013/0102750 | A1 | |
| | | | | claims, examples, figures | | | |
| | | | | EP | 2565219 | A1 | |
| JP | 2007-77393 | A | 29 March 2007 | US | 2007/0041796 | A1 | |
| | | | | claims, examples, figures | | | |
| | | | | EP | 1754725 | A2 | |
| | | | | CN | 1916032 | A | |
| | | | | TW | 200712101 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06345819 A